(19) Europäisches Patentamt
European Patent Office
Office européen des brevets

(11) Veröffentlichungsnummer: **0 021 043**
**B1**

# (12) EUROPÄISCHE PATENTSCHRIFT

(45) Veröffentlichungstag der Patentschrift: **31.03.82**

(51) Int. Cl.³: **C 07 D 265/26**

(21) Anmeldenummer: **80102816.8**

(22) Anmeldetag: **21.05.80**

## (54) Verfahren zur Herstellung von Isatosäureanhydriden.

(30) Priorität: **22.06.79 DE 2925175**

(43) Veröffentlichungstag der Anmeldung: **07.01.81 Patentblatt 81/1**

(45) Bekanntmachung des Hinweises auf die Patenterteilung: **31.03.82 Patentblatt 82/13**

(84) Benannte Vertragsstaaten: **AT BE CH DE FR GB IT LI LU NL SE**

(56) Entgegenhaltungen:
**CH-A-507 279**
**CH-A-512 506**
**CH-A-558 352**
**CH-A5-575 401**
**DD-A-108 298**
**DE-A-2 206 863**
**DE-A1-2 346 308**
**DE-A1-2 556 590**

(73) Patentinhaber: **BASF Aktiengesellschaft, Carl-Bosch-Strasse 38, D-6700 Ludwigshafen (DE)**

(72) Erfinder: **Reissenweber, Gernot, Dr., Pfarrer-Friedrich-Strasse 41, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 021 043 B1

Verfahren zur Herstellung von Isatosäureanhydriden

Die Erfindung betrifft ein neues Verfahren zur Herstellung von Isatosäureanhydriden.

Isatosäureanhydride lassen sich z. B. durch Umsetzung von Anthranilsäuren mit Chlorameisensäureethylester oder Phosgen herstellen [Chem. Ber. 32, 2163—2164 (1898)]. Die hierzu als Ausgangsstoffe benötigten substituierten Anthranilsäuren sind jedoch nur schwer zugänglich, z. B. durch alkalische Oxidation von Isatin-Derivaten:

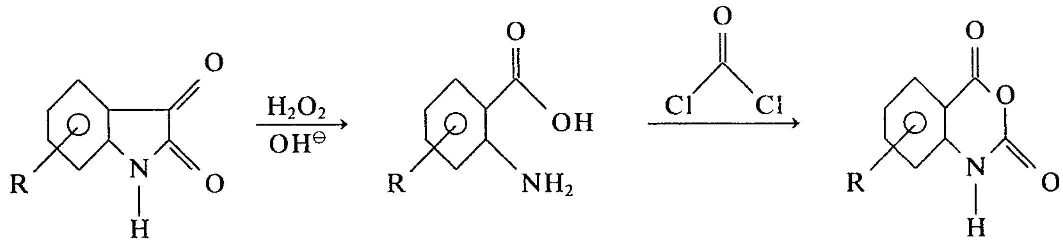


Ein anderer Weg, zu Isatosäureanhydriden zu gelangen, besteht in der Oxidation von substituierten Phthalimiden {J. prakt. Chemie [2] 80, 1–24 (1909)}. Hierbei erhält man jedoch in der Regel Isomerengemische:

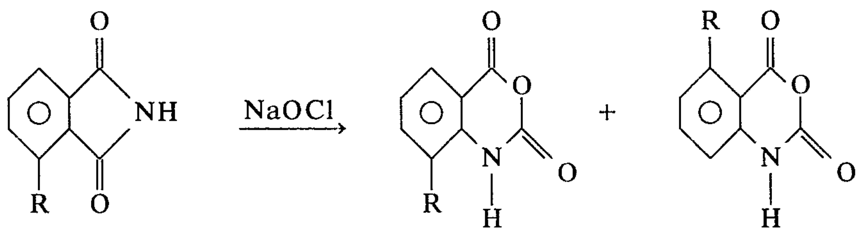


Es ist weiter bekannt, N-phenylsubstituierte Isatine in recht aufwendiger Weise mit Persäuren in die entsprechenden Isatosäureanhydride zu überführen (US-PS 3 238 201). Für am Stickstoff unsubstituierte Isatine galt bisher Chromtrioxid in Eisessig bzw. Essigsäureanhydrid als allein geeignetes Oxidationsmittel. [J. prakt. Chemie 30, 84 (1884); 30, 467 (1884)]. Mischungen von Chromtrioxid und Eisessig bzw. Essigsäureanhydrid können jedoch explodieren. Zudem schafft das toxische Chrom zusätzliche sicherheitstechnische Probleme.

Es wurde nun ein Verfahren zur Herstellung von Isatosäureanhydriden der allgemeinen Formel I

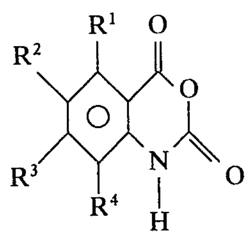

(I)

in welcher

$R^1$, $R^2$, $R^3$ und $R^4$ gleich oder verschieden sind und Wasserstoff, Halogen, Nitro, Alkyl, Alkoxy, Halogenalkyl oder Halogenalkoxy mit jeweils 1 bis 4 Kohlenstoffatomen bedeuten, gefunden, welches darin besteht, daß man Isatine der allgemeinen Formel II

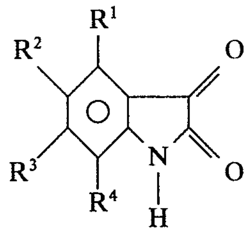

(II)

in welcher

$R^1$, $R^2$, $R^3$ und $R^4$ die oben angegebene Bedeutung haben, mit Wasserstoffperoxid in saurem Medium gegebenenfalls in Gegenwart von anorganischen Säuren oder aromatischen Carbonsäuren umsetzt.

Für das erfindungsgemäße Verfahren löst oder suspendiert man Isatine der allgemeinen Formel II in einer Carbonsäure und tropft eine wäßrige Wasserstoffperoxidlösung zu. Als Lösungsmittel eignen sich aliphatische Carbonsäuren und Halogen-substituierte aliphatische Carbonsäuren, besonders Ameisensäure, Essigsäure, Propionsäure, Chloressigsäure, Trifluoressigsäure, oder auch Mischungen dieser Säuren. Wasserstoffperoxidlösungen können in allen handelsüblichen Konzentrationen verwendet werden, bevorzugt als 30prozentige bzw. 50prozentige wäßrige Lösung. Wasserstoffperoxid wird dabei im molaren Verhältnis 1 : 1 eingesetzt, empfehlenswert ist es jedoch, das Wasserstoffperoxid in geringem Überschuß (Molverhältnis 1 : 1 bis 2 : 1) einzusetzen. Der Temperaturbereich, bei dem die Reaktion durchgeführt werden kann, reicht von 0 bis 100° C, jedoch sind Temperaturen bei Raumtemperatur oder knapp darüber (25 bis 65° C) am günstigsten. Ein Zusatz von anorganischen Säuren oder aromatischen Carbonsäuren wirkt reaktionsbeschleunigend. Bevorzugte Säuren sind beispielsweise Schwefelsäure, Phosphorsäure oder p-Toluolsulfonsäure. Dabei können auf 100 Teile Lösungsmittel 1 bis 50 Teile Säure zugesetzt werden. Besonders vorteilhaft sind 1 bis 5 Teile Säure auf 100 Teile Lösungsmittel.

Die gewünschten Isatosäureanhydride fallen während der Reaktion kristallin an und können so leicht nach den üblichen Methoden, z. B. durch Filtrieren oder Zentrifugieren, isoliert werden.

Im Vergleich zu den bekannten Verfahren liefert das erfindungsgemäße Verfahren, ausgehend von leicht zugänglichen Ausgangsstoffen, in überraschend einfacher Weise und auf einem sehr wirtschaftlichen Weg die gewünschten Verbindungen in hoher Ausbeute und Reinheit.

Isatosäureanhydride sind wertvolle Zwischenprodukte zur Herstellung von Arznei- und Pflanzenschutzmitteln. So erhält man beispielsweise durch Umsetzung von Isatosäureanhydriden mit Isopropylamin die entsprechenden Anthranilsäureisopropylamide, aus denen 2,1,3-Thiadiazin-4-on-2,2-dioxidderivate hergestellt werden können, die bekannte Pflanzenschutzmittel sind (DE-OS 2 710 382).

## Beispiel 1

16,1 Teile 7-Methylisatin werden in 150 Teilen Essigsäure und 2,5 Teilen konzentrierter Schwefelsäure suspendiert. Bei 35°C tropft man innerhalb von 10 Minuten 15 Teile 30prozentige Wasserstoffperoxidlösung zu und rührt die Suspension zwei Stunden bei 60° C. Nach dem Erkalten wird der Niederschlag abgesaugt und mit Wasser gewaschen. Man erhält 14,2 Teile 8-Methylisatosäureanhydrid (Fp 236 bis 238° C; Zersetzung).

Dasselbe Ergebnis erhält man, wenn man die Essigsäure durch ein Gemisch Essigsäure/Chloressigsäure 2 : 1 oder Essigsäure/Trifluoressigsäure 2 : 1 ersetzt.

## Beispiel 2

14,7 Teile Isatin werden in 80 Teilen Ameisensäure suspendiert. Unter leichter Kühlung werden 20 Teile 30prozentige Wasserstoffperoxidlösung zugetropft. Anschließend wird 60 Minuten bei 25°C gerührt. Danach wird wie üblich aufgearbeitet. Man erhält 13,2 Teile Isatosäureanhydrid (Fp 252 bis 253° C; Zersetzung).

## Beispiel 3

41 Teile 6-Fluorisatin werden in 500 Teilen Eisessig und 15 Teilen konzentrierter Schwefelsäure suspendiert und bei 30°C mit 100 Teilen 30prozentiger Wasserstoffperoxidlösung versetzt. Man beobachtet eine leicht exotherme Reaktion, wobei bei 40 bis 45° C zwischenzeitlich eine klare Lösung entsteht. Man läßt die Temperatur nicht über 50°C steigen und kühlt nach einer Stunde auf Raumtemperatur ab. Der Niederschlag wird abgesaugt und gewaschen. Man erhält 38 Teile 7-Fluorisatosäureanhydrid (Fp 229 bis 231° C; Zersetzung).

## Beispiel 4

Zu einer auf 40°C vorgewärmten Lösung aus 900 Teilen Essigsäure und 15 Teilen Schwefelsäure werden 108 Teile 5,7-Dichlorisatin und danach langsam 80 Teile 50prozentige Wasserstoffperoxidlösung gegeben. Man läßt die Temperatur bis auf 65° C ansteigen. Die Suspension geht in Lösung und nach wenigen Minuten beginnt das gewünschte Produkt, aus der warmen Lösung auszufallen. Man rührt eine Stunde nach und erhält nach dem Abkühlen 103 Teile 6,8-Dichlorisatosäureanhydrid (Fp 254 bis 256° C; Zersetzung).

Beispiel 5

16,1 Teile 7-Methylisatin werden in einer Mischung aus 120 Teilen Eisessig und 10 Teilen Ameisensäure suspendiert und bei 50° C mit 15 Teilen 30prozentigem Wasserstoffperoxid versetzt. Man rührt 2,5 Stunden bei 50° C und erhält nach dem Abkühlen 12,6 Teile 8-Methylisatorsäureanhydrid (Fp 237 bis 239° C; Zersetzung).

Beispiel 6

43 Teile 7-Trifluormethylisatin werden in 130 Teilen Eisessig und 2,5 Teilen Schwefelsäure suspendiert und bei 40° C mit 25 Teilen 30prozentiger Wasserstoffperoxidlösung versetzt. Nach einer Stunde Rühren bei nicht über 55° C erhält man 38 Teile 8-Trifluormethylisatosäureanhydrid (Fp 184 bis 186° C; Zersetzung).

Auf gleiche Weise wurden dargestellt:

| | | |
|---|---|---|
| 6-Brom-8-methylisatosäureanhydrid | (Fp > 270° C) | 80% Ausbeute |
| 6-Fluorisatosäureanhydrid | (Fp 265—268° C; Zersetzung) | 83% Ausbeute |
| 6-Bromisatosäureanhydrid | (Fp 270—275° C; Zersetzung) | 83% Ausbeute |
| 6-Nitroisatosäureanhydrid | (Fp 224—232° C; Zersetzung) | 80% Ausbeute |
| 8-Chlorisatosäureanhydrid | (Fp 210—215° C; Zersetzung) | 85% Ausbeute |
| 5-Methyl-8-methoxyisatosäureanhydrid | (Fp 235—242° C; Zersetzung) | 75% Ausbeute |

**Patentansprüche**

1. Verfahren zur Herstellung von Isatosäureanhydriden der allgemeinen Formel I

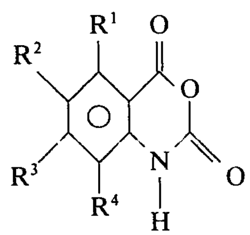

(I)

in welcher
$R^1$, $R^2$, $R^3$ und $R^4$ gleich oder verschieden sind und Wasserstoff, Halogen, Nitro, Alkyl, Alkoxy, Halogenalkyl oder Halogenalkoxy mit jeweils 1 bis 4 Kohlenstoffatomen bedeuten, dadurch gekennzeichnet, daß man Isatine der allgemeinen Formel II

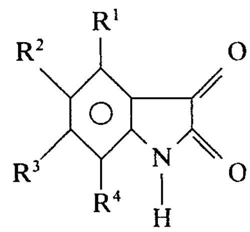

(II)

in welcher
$R^1$, $R^2$, $R^3$ und $R^4$ die oben angegebene Bedeutung haben, mit Wasserstoffperoxid in saurem Medium, gegebenenfalls in Gegenwart von anorganischen Säuren oder aromatischen Carbonsäuren umsetzt.

2. Verfahren gemäß Anspruch 1, dadurch gekennzeichnet, daß man die Umsetzung in Gegenwart von zusätzlich 1 bis 50, vorzugsweise 1 bis 5 Teilen einer anorganischen Säure pro 100 Teile Lösungsmittel durchführt.

## Claims

1. A. process for the preparation of isatoic anhydrides of the general formula I

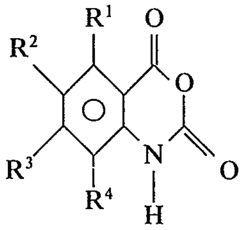

(I)

where

$R^1$, $R^2$, $R^3$ and $R^4$ are identical or different and each is hydrogen, halogen or nitro, or alkyl, alkoxy, haloalkyl or haloalkoxy, each having 1 to 4 carbon atoms, characterized in that an isatin of the general formula II

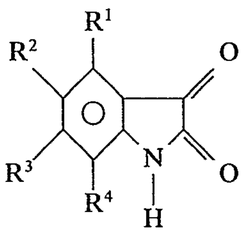

(II)

where

$R^1$, $R^2$, $R^3$ and $R^4$ have the above meanings, is reacted with hydrogen peroxide in an acid medium in the presence or absence of an inorganic acid or an aromatic carboxylic acid.

2. A process as claimed in claim 1, characterized in that the reaction is carried out in the presence of from 1 to 50, preferably from 1 to 5, parts of an inorganic acid per 100 parts of solvent.

## Revendications

1. Procédé de préparation d'anhydrides isatoiques de formule générale I

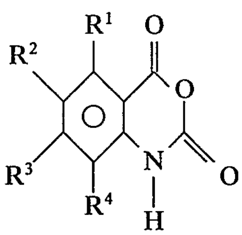

(I)

dans laquelle

$R^1$, $R^2$, $R^3$ et $R^4$ sont identiques ou différents et représentent hydrogène, halogène, nitro, alkyle, alcoxy, halogénoalkyle ou halogéno-alcoxy portant dans chaque cas 1 à 4 atomes de carbone, caractérisé par le fait que l'on fait réagir des isatines de formule générale II

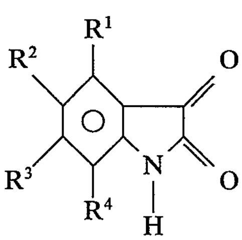

(II)

dans laquelle

$R^1$, $R^2$, $R^3$ et $R^4$ ont les significations données plus haut, avec du peroxyde d'hydrogène en milieu acide, le cas échéant en présence d'acides inorganiques ou d'acides carboxyliques aromatiques.

2. Procédé selon la revendication 1, dans lequel on effectue la réaction avec addition supplémentaire de 1 à 50 parties, de préférence 1 à 5, d'un acide inorganique pour 100 parties de solvant.